# EUROPEAN PATENT SPECIFICATION

(11) **EP 4 205 798 B1**
(45) Date of publication and mention of the grant of the patent: **23.04.2025**
(21) Application number: 21218121.8
(22) Date of filing: 29.12.2021
(51) Int. Cl.: A61N 1/05, A61N 1/32, A61N 1/36, A61N 1/372, A61N 1/378, A61N 1/40, A61N 2/00, A61N 2/02

(54) **IMPLANT, STIMULATION SYSTEM AND METHOD OF MANUFACTURING AN IMPLANT**
IMPLANTAT, STIMULATIONSSYSTEM UND VERFAHREN ZUR HERSTELLUNG EINES IMPLANTATS
IMPLANT, SYSTÈME DE STIMULATION ET PROCÉDÉ DE FABRICATION D'UN IMPLANT

(43) Date of publication of application: 05.07.2023
(73) Proprietor: Gottfried Wilhelm Leibniz Universität Hannover, 30167 Hannover (DE); Medizinische Hochschule Hannover, 30625 Hannover (DE)
(72) Inventor: Krezdorn, Nicco, 30169 Hannover (DE); Höltje, Kai, 30419 Hannover (DE); Müller, Marc, 30449 Hannover (DE); Glasmacher, Birgit, 30419 Hannover (DE)
(74) Representative: Kröncke, Rolf

(56) References cited:
- WO-A1-2020/206332
- US-A1- 2009 062 886
- US-A1- 2013 317 279
- US-A1- 2017 265 927
- US-A1- 2018 036 532

## Description

The invention relates to an implant which is arranged for implanting in the body of a patient, the implant being arranged to emit mechanical and/or electrical stimulation signals to the tissue of the patient surrounding the implanted implant. The invention also relates to a stimulation system for stimulating tissue of a patient using such an implant, and a method of manufacturing such an implant. The implant is suitable for various applications in stimulating tissue of a patient. In the following, muscle stimulation will be primarily discussed, but other applications for stimulating tissue will also be explained.

In the case of e.g. traumatic severing of motor nerve fibres, paralysis of target muscles occurs because the electrical signal can no longer be transmitted to the muscle via the nerve fibre. In the absence of regenerative capacity or anatomically proximal transection, permanent muscle atrophy may occur due to the lack of innervation, resulting in severely limited or complete loss of function of the respective muscles. To counteract this, there are already proposals such as transcutaneous electrical nerve stimulation (TENS), which, however, only allows uncontrolled stimulation of the superficial muscles to prevent muscle atrophy after paralysis. The results of this appear to be in need of improvement. Other proposals for direct muscle stimulation via the insertion of individual electrodes into the patient cannot be usefully applied in practice due to the limited surface area and the resulting muscle necrosis.

WO 2020/206332 A1 discloses magnetoelectric data and power to miniature biode-vices with tunable amplitude and waveform. US 2009/0062886 A1 discloses systems and methods for delivering electrical energy in the human body.

It is therefore an object of the invention to provide improved technical means for stimulating tissue of a patient by means of mechanical and/or electrical stimulation signals, leading to more satisfactory results. Furthermore, a method for manufacturing such an implant is needed.

This task is solved by an implant according to claim 1. The implant is arranged for implanting in the body of a patient, the implant being arranged to emit mechanical and/or electrical stimulation signals to the tissue of the patient surrounding the implanted implant, wherein the implant is shaped as a thin, sheet-like body which is formed from one material layer or a plurality of material layers lying one on top of the other, of which at least a first material layer is ferromagnetically functionalized. The invention has the advantage that the implant, due to its construction as a thin sheet-like body, can be easily arranged at the positions to be stimulated in the body of a patient, for example, in the case of a paralyzed muscle it can be surgically applied directly to the muscle, or in the case of a bone it can be directly applied to the bone. For example, the implant may be implanted directly under the fascia on the muscle.

Advantageously, at least a first material layer of the thin sheet-like body is ferromagnetically functionalized, i.e. this material layer reacts to magnetic fields and can thus be mechanically deformed by applying magnetic fields. This in turn has the advantage that for the generation and control of the desired stimulation signals in the patient's body, no conductive connection to an extracorporeally arranged stimulation unit has to be established. It is also not necessary to implant an additional stimulation unit in the patient. Rather, by selectively generating magnetic fields outside the body in the vicinity of the location of the body at which the implant is implanted in the body, a desired and, above all, targeted deformation of the thin sheet-like body can be induced due to the ferromagnetic properties of the first material layer.

In an advantageous embodiment, the implant or its thin sheet-like body can be made biocompatible.

Such a thin sheet-like body is characterized by having a relatively large area compared to the material thickness of the body. For example, the planar extent (surface area) of the thin sheet-like body relative to the thickness may have a value of at least 50 mm. The thin sheet-like body may have a planar extent of, for example, at least 0.5 cm² or at least 1 cm², but may also be designed to be even larger, depending on the application. The thickness of the thin sheet-like body may, for example, be less than 1 mm, in particular less than 0.5 mm. Thicknesses in the range of 300 µm or less are advantageous, for example. The thin sheet-like body may have a flat or planar shape. The thin sheet-like body may be flexible, such that it can be elastically or plastically deformed into a desired shape required at the implantation site.

According to an advantageous embodiment of the invention, the implant is flexibly placeable around at least one internal body structure of the patient. Accordingly, the implant is elastically or plastically deformable, whereby the shape once adopted can also be retained independently. The implant can then be specifically arranged at the desired location in the patient during an operation by placing it at least partially around the internal body structure, for example a muscle, a nerve or a bone. In doing so, the implant does not have to completely surround the internal body structure of the patient, even a partial surrounding may be sufficient for a desired stimulation in many cases.

According to the invention at least the first material layer of the thin sheet-like body is formed as a nonwoven layer of micro- and/or nanofibers produced by electrospinning.

According to an advantageous embodiment of the invention, several or all material layers of the thin sheet-like body are formed as nonwoven layers, in particular as a nonwoven layer of nanofibers. In this way, a biocompatible and sufficiently flexible thin planar body can be produced in a simple and inexpensive manner. In this context, a nonwoven is understood to be any type of fabric-like material made of fibers that adhere together, like a fibrous web or mat of fibers. The individual fibers of the nonwoven can be formed as micro- and/or nanofibers. Nanofibers are fibers with a diameter less than 1000 Nanometer (nm), or a diameter less than 400 nm.

According to an advantageous embodiment of the invention, several or all of the nonwoven layers are produced by electrospinning. This allows a very precise, simple and cost-effective production of the nonwoven layers.

The implant can also be manufactured patient-specific with regard to its functionality and/or dimensions.

According to an advantageous embodiment of the invention, the thin sheet-like body has at least a second material layer that is piezoelectrically functionalized. This has the advantage that, due to the piezoelectric effect, electrical stimulation signals can be emitted by the second material layer when the thin sheet-like body is deformed, which signals can be emitted, for example, directly as electrical stimulation signals to the tissue of the patient surrounding the implant. The deformation of the second material layer required for this purpose can be generated by selectively applying magnetic fields to the first material layer, which is ferromagnetic. In this case, the second material layer has to be firmly connected to the first material layer, either directly or via at least one intermediate layer.

The piezoelectrically functionalized second material layer may be made, for example, of a polymer, such as PVDF (polyvinylidene difluoride), PVDF-TrFE, or a similar semi-crystalline thermoplastic fluoroplastic.

According to an advantageous embodiment of the invention, the second material layer is connected to the first material layer via a third material layer, which is formed as an insulation layer. This has the advantage that a targeted delivery of the electrical stimulation signals from the second material layer is only permitted in one direction, namely directly to the tissue of the patient on which the implant is placed. Accordingly, no electrical stimulation signals are emitted on the opposite side, i.e. via the first material layer. In this embodiment, the thin sheet-like body thus has a three-layer structure, where on one side is the second material layer, on the other side is the first material layer, and in between is the third material layer. In addition, a further insulation layer can be arranged on the first material layer, so that the first material layer is then arranged between two insulation layers. The mentioned insulation layers, i.e. in particular the third material layer, may be formed as a non-woven layer or as a foil.

According to an advantageous embodiment of the invention, the second material layer is arranged for emitting piezoelectrically generated electrical stimulation signals to the surrounding tissue simultaneously and/or sequentially at a plurality of surface locations of the thin sheet-like body. This has the advantage that a precisely controlled stimulation of the surrounding tissue by the implant can be generated by selectively generating magnetic fields outside the body. Accordingly, an electrical stimulation signal, i.e. a voltage, is emitted at that point of the second material layer which corresponds to a position where the magnetic fields trigger a deformation of the first material layer.

According to an advantageous embodiment of the invention, the first material layer is ferromagnetically functionalized by introducing ferromagnetic nanoparticles into the first material layer. This has the advantage that the manufacturing process of the first material layer can be realized very simply and reliably, namely by the aforementioned electrospinning process in which the ferromagnetic nanoparticles are also fed in. For example, ferromagnetic nanoparticles with a particle size <20 nm can be used, or with a particle size <10 nm. For example, ferromagnetic nanoparticles of magnetite, iron and/or neodymium are suitable. Alternatively or in addition to the introduction of ferromagnetic particles, a ferromagnetic coating of the first material layer can also be implemented.

According to an advantageous embodiment of the invention, the implant is arranged to perform muscle stimulation, brain stimulation, nerve stimulation and/or bone stimulation by mechanical and/or electrical stimulation signals emitted by the implant. Accordingly, the implant can be used very universally for a wide variety of types of stimulation applications. For example, the implant may be implanted on a muscle, such as under the fascia, or on a nerve, such as the optic nerve, to restore a patient's vision, or on a bone to stimulate bone growth by the appropriate stimulation signals, such as to heal a fracture.

The aforementioned task is also solved by a transcutaneous stimulation system for stimulating tissue of a patient by means of mechanical and/or electrical stimulation signals within the body, wherein the stimulation system comprises an implant of the aforementioned type and a stimulation unit to be arranged outside the body of the patient, comprising a magnetic field generating component adapted to emit magnetic fields in a controlled manner at one or more locations of the stimulation unit or throughtout the patient's body. The advantages explained above can also be realized by such a stimulation system. In this respect, the stimulation unit can be applied, for example, only when required, i.e. placed on the patient's body only when required, in order to stimulate the implant within the body to emit mechanical and/or electrical stimulation signals.

For example, the stimulation unit may have one or more electromagnets for generating the magnetic fields, for example in the form of a matrix-like arrangement of a plurality of electromagnets.

The stimulation unit may be computer controlled, for example, so that a particular stimulation program, i.e. a stimulation sequence, may be performed in a program-controlled manner. The stimulation unit may also be connected, for example via a suitable interface, to another part of the patient's body, for example to the brain and/or nerves, so that signals generated by the patient's brain and/or nerves can be converted in the stimulation unit into control signals for controlling electromagnets of the stimulation unit, and magnetic fields are accordingly selectively delivered by the stimulation unit to trigger electrical and/or mechanical stimulation signals within the body through the implant.

The aforementioned task is also solved by a method according to claim 10 for producing an implant of the type explained above. This has the advantage that the fiber structure, layer thickness and the desired piezoelectric effect of the piezoelectrically functionalized material layer can be easily adapted depending on the desired application.

The invention thus permits stimulation of tissue of a patient over a wide area, for example muscle tissue, without damaging surrounding tissue by skin punctures or the like. In addition, the implant according to the invention can be used very flexibly for different modes of stimulation.

The advantages lie in the regaining of motor abilities after partial or complete paralysis of muscles and extremities. Complicated plastic-surgical motor replacement plastics, with which only adequate results can be achieved, would become superfluous. Expensive, complex, error-prone external prostheses that are often poorly accepted by patients are also no longer necessary.

In addition to the actual construction of the implant, the form of the therapy must also be mentioned. On the one hand, this can be carried out over a wide area of the patient's body and not only at certain points, thus exceeding the state of the art. In addition, the introduction of the necessary energy in the patient's body is non-invasive, which offers extensive advantages over established solutions (comfort, infections, necroses). At the same time, the generation of the mechanical and/or electrical stimulation signals by the implant is very variable in amplitude and frequency.

The entire system, consisting of the implant as a receiver unit (multilayer) and the stimulation unit for control, is designed for long-term use. The receiver unit is explicitly designed for long-term implantation.

The invention is explained in more detail below with reference to exemplary embodiments of the invention using drawings.

The drawings show in
Figure 1 a stimulation system in schematic representation,
Figure 2 a multi-layered structure of a thin sheet-like body of the implant,
Figure 3 the arrangement of the implant on an internal body structure of a patient.

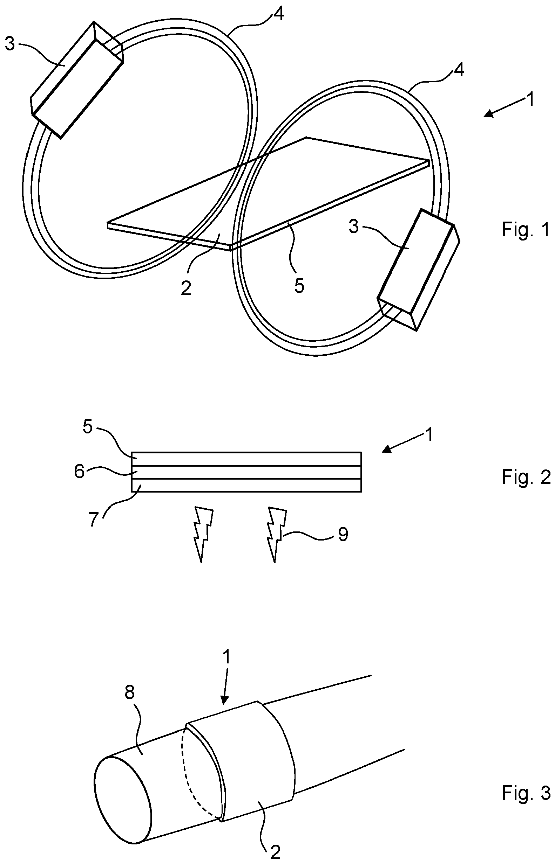

Figure 1 shows a stimulation system comprising an implant 1 and a stimulation unit 3. The implant 1 is adapted to be implanted in the body of a patient. As can be seen, the implant 1 is formed as a thin sheet-like body 2. The thin sheet-like body 2 has one or more material layers, wherein at least a first material layer 5 is present which is ferromagnetically functionalized.

The stimulation unit 3 is arranged to emit controlled magnetic fields 4 at one or more locations of the stimulation unit 3, for example by means of electromagnets arranged on or in the stimulation unit 3. These magnetic fields 4 are emitted through the tissue of a patient to the implant 1 implanted in the patient's body, where they cause local deformations to the first material layer 5 due to its ferromagnetic properties. The local deformations can, for example, deliver mechanical stimulation signals to the underlying and surrounding tissue of the patient in a directed manner. Multiple stimulation units 3 can be combined for coordination of complex movements of an array of tissue equipped with implants.

Figure 2 shows a multi-layered version of the implant 1 or its thin sheet-like body 2 in a lateral sectional view. The multi-layer structure of the thin sheet-like body 2 consists of the first material layer 5, which is ferromagnetically functionalised, a third material layer 6 in the form of an insulation layer which is arranged underneath first material layer 5 and a second material layer 7 which is piezoelectrically functionalised and is arranged underneath third material layer 6. In each case, adjacent material layers are firmly connected to one another. If deformations are applied to the second material layer 7, for example due to a magnetically induced deformation of the first material layer 5, the second material layer 7 emits electrical stimulation signals 9.

Figure 3 shows the application of the implant 1 according to the invention to an internal body structure 8 of a patient, for example to a muscle. The implant 1 is placed around the muscle, in particular below the fascia. The implant 1 in contact with the muscle 8 can then transmit electrical stimulation signals 9 directly to the muscle 8, thereby stimulating it to contract.

The magnetic fields emitted by the stimulation unit 3 are transmitted through the patient's skin to the implant 1, where they cause the corresponding mechanical deformations and, accordingly, the emission of the electrical stimulation signals. The stimulation unit 3 can always be arranged outside the patient's body.

The implant 1 can grow in completely inside the patient's body. This reduces the risk of infection, as no permanent openings of the skin are necessary for the power supply.

### Reference list

- 1: Implant
- 2: Thin sheet-like body
- 3: Stimulation unit
- 4: Magnetic field
- 5: First material layer
- 6: Third material layer
- 7: Second material layer
- 8: Internal body structure
- 9: Electrical stimulation signals

## Claims

1. Implant (1) which is arranged for implanting in the body of a patient, the implant being arranged to emit mechanical and/or electrical stimulation signals (9) to the tissue of the patient surrounding the implanted implant (1), wherein the implant (1) is shaped as a thin, sheet-like body (2) which is formed from one material layer (5) or a plurality of material layers lying one on top of the other, of which at least a first material layer (5) is ferromagnetically functionalized, wherein at least the first material layer (5) of the thin sheet-like body (2) is formed as a nonwoven layer of micro- and/or nanofibers produced by electrospinning, **characterized in that** the first material layer (5) is ferromagnetically functionalized by introducing ferromagnetic nanoparticles into the first material layer (5) by feeding in the ferromagnetic nanoparticles in the electrospinning process.

2. Implant (1) according to claim 1, **characterized in that** the implant (1) is flexibly placeable around at least one internal body structure (8) of the patient.

3. Implant (1) according to one of the preceding claims, **characterized in that** several or all material layers of the thin sheet-like body (2) are formed as nonwoven layers, in particular as a nonwoven layer of micro- and/or nanofibers.

4. Implant (1) according to claim 3, **characterized in that** several or all of the nonwoven layers are produced by electrospinning.

5. Implant (1) according to any one of the preceding claims, **characterized in that** the thin sheet-like body (2) has at least a second material layer (7) that is piezoelectrically functionalized.

6. Implant (1) according to claim 5, **characterized in that** the second material layer (7) is connected to the first material layer (5) via a third material layer (6), which is formed as an insulation layer.

7. Implant (1) according to claim 5 or 6, **characterized in that** the second material layer (7) is arranged for emitting piezoelectrically generated electrical stimulation signals to the surrounding tissue simultaneously and/or sequentially at a plurality of surface locations of the thin sheet-like body (2).

8. Implant (1) according to any one of the preceding claims, **characterized in that** the implant (1) is arranged to perform muscle stimulation, brain stimulation, nerve stimulation and/or bone stimulation by mechanical and/or electrical stimulation signals (9) emitted by the implant (1).

9. Stimulation system for stimulating tissue of a patient by means of mechanical and/or electrical stimulation signals (9) within the body, **characterized in that** the stimulation system comprises an implant (1) according to one of the preceding claims and a stimulation unit (3) to be arranged outside the body of the patient, comprising a magnetic field (4) generating component adapted to emit magnetic fields (4) in a controlled manner at one or more locations of the stimulation unit (3) or throughout the patient's body.

10. Method of manufacturing an implant (1) according to any one of claims 1 to 9, wherein at least the first material layer of the thin sheet-like body (2) is produced as non-woven layer by electrospinning, **characterized in that** the first material layer (5) is ferromagnetically functionalized by introducing ferromagnetic nanoparticles into the first material layer (5) by feeding in the ferromagnetic nanoparticles in the electrospinning process.

## Patentansprüche

1. Implantat (1), das zur Implantation in den Körper eines Patienten eingerichtet ist, wobei das Implantat eingerichtet ist, um mechanische und/oder elektrische Stimulationssignale (9) an das das implantierte Implantat (1) umgebende Gewebe des Patienten abzugeben, wobei das Implantat (1) als dünner, flächiger Körper (2) geformt ist, der aus einer Materialschicht (5) oder mehreren übereinanderliegenden Materialschichten gebildet ist, von denen mindestens eine erste Materialschicht (5) ferromagnetisch funktionalisiert ist, wobei zumindest die erste Materialschicht (5) des dünnen flächigen Körpers (2) als eine durch Elektrospinnen hergestellte Vliesschicht aus Mikro- und/oder Nanofasern ausgebildet ist, **dadurch gekennzeichnet, dass** die erste Materialschicht (5) durch Einbringen ferromagnetischer Nanopartikel in die erste Materialschicht (5) durch Zuführen der ferromagnetischen Nanopartikel im Elektrospinnverfahren ferromagnetisch funktionalisiert ist.

2. Implantat (1) nach Anspruch 1, **dadurch gekennzeichnet, dass** das Implantat (1) flexibel um mindestens eine innere Körperstruktur (8) des Patienten platzierbar ist.

3. Implantat (1) nach einem der vorhergehenden Ansprüche, **dadurch gekennzeichnet, dass** mehrere oder alle Materialschichten des dünnen flächigen Körpers (2) als Vliesschichten, insbesondere als eine Vliesschicht aus Mikro- und/oder Nanofasern, ausgebildet sind.

4. Implantat (1) nach Anspruch 3, **dadurch gekennzeichnet, dass** mehrere oder alle der Vliesschichten durch Elektrospinnen hergestellt sind.

5. Implantat (1) nach einem der vorhergehenden Ansprüche, **dadurch gekennzeichnet, dass** der dünne flächige Körper (2) mindestens eine zweite Materialschicht (7) aufweist, die piezoelektrisch funktionalisiert ist.

6. Implantat (1) nach Anspruch 5, **dadurch gekennzeichnet, dass** die zweite Materialschicht (7) mit der ersten Materialschicht (5) über eine dritte Materialschicht (6) verbunden ist, die als Isolationsschicht ausgebildet ist.

7. Implantat (1) nach Anspruch 5 oder 6, **dadurch gekennzeichnet, dass** die zweite Materialschicht (7) so angeordnet ist, dass sie an einer Vielzahl von Oberflächenstellen des dünnen flächigen Körpers (2) gleichzeitig und/oder nacheinander piezoelektrisch erzeugte elektrische Stimulationssignale an das umgebende Gewebe abgibt.

8. Implantat (1) nach einem der vorhergehenden Ansprüche, **dadurch gekennzeichnet, dass** das Implantat (1) so angeordnet ist, dass es eine Muskelstimulation, eine Hirnstimulation, eine Nervenstimulation und/oder eine Knochenstimulation durch mechanische und/oder elektrische Stimulationssignale (9) durchführt, die von dem Implantat (1) abgegeben werden.

9. Stimulationssystem zur Stimulation von Gewebe eines Patienten mittels mechanischer und/oder elektrischer Stimulationssignale (9) innerhalb des Körpers, **dadurch gekennzeichnet, dass** das Stimulationssystem ein Implantat (1) nach einem der vorhergehenden Ansprüche und eine außerhalb des Körpers des Patienten anzuordnende Stimulationseinheit (3) umfasst, die eine ein Magnetfeld (4) erzeugende Komponente aufweist, die geeignet ist, Magnetfelder (4) in kontrollierter Weise an einer oder mehreren Stellen der Stimulationseinheit (3) oder im gesamten Körper des Patienten abzugeben.

10. Verfahren zur Herstellung eines Implantats (1) nach einem der Ansprüche 1 bis 9, wobei zumindest die erste Materialschicht des dünnen flächigen Körpers (2) als Vliesschicht durch Elektrospinnen hergestellt wird, **dadurch gekennzeichnet, dass** die erste Materialschicht (5) ferromagnetisch funktionalisiert wird, indem ferromagnetische Nanopartikel in die erste Materialschicht (5) eingebracht werden, indem die ferromagnetischen Nanopartikel im Elektrospinnverfahren zugeführt werden.

## Revendications

1. Implant (1) conçu pour être implanté dans le corps d'un patient, l'implant étant conçu pour émettre des signaux de stimulation mécanique et/ou électrique (9) au tissu du patient entourant l'implant (1) implanté, l'implant (1) ayant la forme d'un corps mince en forme de feuille (2) formé d'une couche de matériau (5) ou d'une pluralité de couches de matériau superposées, dont au moins une première couche de matériau (5) est fonctionnalisée ferromagnétiquement, au moins la première couche de matériau (5) du corps mince en forme de feuille (2) étant formée comme une couche non tissée de microfibres et/ou de nanofibres produites par électrofilage,
**caractérisé en ce que** la première couche de matériau (5) est fonctionnalisée ferromagnétiquement par l'introduction de nanoparticules ferromagnétiques dans la première couche de matériau (5) par approvisionnement des nanoparticules ferromagnétiques dans le processus d'électrofilage.

2. Implant (1) selon la revendication 1,
**caractérisé en ce que** l'implant (1) peut être placé de manière flexible autour d'au moins une structure interne (8) du corps du patient.

3. Implant (1) selon l'une des revendications précédentes,
**caractérisé en ce que** plusieurs ou toutes les couches de matériau du corps mince en forme de feuille (2) sont formées comme des couches non tissées, en particulier comme une couche non tissée de microfibres et/ou de nanofibres.

4. Implant (1) selon la revendication 3,
**caractérisé en ce que** plusieurs ou toutes les couches non tissées sont produites par électrofilage.

5. Implant (1) selon l'une des revendications précédentes,
**caractérisé en ce que** le corps mince en forme de feuille (2) comprend au moins une deuxième couche de matériau (7) fonctionnalisée piézoélectriquement.

6. Implant (1) selon la revendication 5,
**caractérisé en ce que** la deuxième couche de matériau (7) est reliée à la première couche de matériau (5) par l'intermédiaire d'une troisième couche de matériau (6) qui est formée comme une couche d'isolation.

7. Implant (1) selon la revendication 5 ou 6,
**caractérisé en ce que** la deuxième couche de matériau (7) est conçue pour émettre des signaux de stimulation électrique générés piézoélectriquement vers le tissu environnant, simultanément et/ou séquentiellement, à plusieurs endroits de la surface du corps mince en forme de feuille (2).

8. Implant (1) selon l'une des revendications précédentes,
**caractérisé en ce que** l'implant (1) est conçu pour effectuer une stimulation musculaire, une stimulation cérébrale, une stimulation nerveuse et/ou une stimulation osseuse par des signaux de stimulation mécanique et/ou électrique (9) émis par l'implant (1).

9. Système de stimulation pour stimuler le tissu d'un patient au moyen de signaux de stimulation mécanique et/ou électrique (9) à l'intérieur du corps, **caractérisé en ce que** le système de stimulation comprend un implant (1) selon l'une des revendications précédentes et une unité de stimulation (3) à disposer à l'extérieur du corps du patient, comprenant un composant générateur de champ magnétique (4) adapté pour émettre des champs magnétiques (4) de manière contrôlée à un ou plusieurs endroits de l'unité de stimulation (3) ou dans l'ensemble du corps du patient.

10. Procédé de fabrication d'un implant (1) selon l'une des revendications 1 à 9,
dans lequel au moins la première couche de matériau du corps mince en forme de feuille (2) est réalisée comme une couche non tissée par électrofilage,
**caractérisé en ce que** la première couche de matériau (5) est fonctionnalisée ferromagnétiquement par l'introduction de nanoparticules ferromagnétiques dans la première couche de matériau (5) par approvisionnement des nanoparticules ferromagnétiques dans le processus d'électrofilage.
